# EUROPEAN PATENT APPLICATION

(11) **EP 3 564 385 A1**
(43) Date of publication of application: **06.11.2019**
(21) Application number: 17888761.8
(22) Date of filing: 26.12.2017
(51) Int. Cl.: C12Q 1/02, C12N 5/071, C12N 5/10

(54) **EVALUATION METHOD AND SELECTION METHOD FOR INDUCED PLURIPOTENT STEM CELLS, AND PRODUCTION METHOD FOR INDUCED PLURIPOTENT STEM CELLS**

(30) Priority: 27.12.2016 JP 2016252672
(71) Applicant: Sumitomo Chemical Company, Limited, Tokyo 104-8260 (JP); Kyoto University, Kyoto-shi, Kyoto 606-8501 (JP)
(72) Inventor: SASAKI, Katsunori, Chuo-ku Tokyo 104-8260 (JP); YAMADA, Sachiko, Osaka-shi Osaka 554-8558 (JP); SAITO, Koichi, Osaka-shi Osaka 554-8558 (JP); KANEKO, Shin, Kyoto-shi Kyoto 606-8501 (JP); KITAYAMA, Shuichi, Kyoto-shi Kyoto 606-8501 (JP); OKITA, Keisuke, Kyoto-shi Kyoto 606-8501 (JP)
(74) Representative: J A Kemp LLP
(86) International application number: PCT/JP2017/046755
(87) International publication number: WO 2018/124118

(57) **Abstract**

The present invention provides a method for evaluating induced pluripotent stem cells, containing a step of providing cultured induced pluripotent stem cells, a step of measuring spontaneous frequency of an organelle having an abnormal nucleic acid structure in the provided induced pluripotent stem cells, and a step of identifying induced pluripotent stem cells having a spontaneous frequency of not more than a reference value, and induced pluripotent stem cells having the spontaneous frequency exceeding the reference value, as well as a method for selecting induced pluripotent stem cells further containing a step of selecting induced pluripotent stem cells having the spontaneous frequency of not more than the reference value. Furthermore, the present invention provides a production method of induced pluripotent stem cells, containing a step of providing established and cultured induced pluripotent stem cells, a step of measuring a spontaneous frequency of an organelle having an abnormal nucleic acid structure in the provided induced pluripotent stem cells, a step of identifying induced pluripotent stem cells having the spontaneous frequency of not more than a reference value, and induced pluripotent stem cells having the spontaneous frequency exceeding the reference value, and a step of selecting induced pluripotent stem cells having the spontaneous frequency of not more than the reference value.

## Description

### [Technical Field]

The present invention relates to an evaluation method and a selection method of induced pluripotent stem cells, and a production method of induced pluripotent stem cells.

### [Background Art]

Induced pluripotent stem cell (iPS cell) is a pluripotent stem cell produced by artificially manipulating a somatic cell. Examples thereof include iPS cells established by Yamanaka et al. from mouse cells in 2006 and from human cells in 2007. Unlike embryonic stem cells (ES cells), induced pluripotent stem cells are produced from somatic cells of individual organisms without using fertilized eggs. Thus, avoidance of ethical problems of concern that occur when using ES cells and rejection during transplantation is expected.

Therefore, the application of differentiated cells derived from induced pluripotent stem cells to regenerative medicine is attracting much attention.

When performing regenerative medicine using induced pluripotent stem cells, there is a concern about tumorigenesis originated from graft cells. The cause of tumorigenesis is considered to be related to the quality of the induced pluripotent stem cells to be used, as well as the contamination with undifferentiated cells.

It has been reported that a cell population that has changed advantageously for cell proliferation due to changes in the genomic structure may emerge, for example, in the step of culturing established induced pluripotent stem cells in the process of producing the induced pluripotent stem cells. Changes in the genomic structure that act advantageously for cell proliferation are closely related to tumorigenesis. Therefore, the cell population that underwent such changes becomes a risk factor in the application of induced pluripotent stem cells to regenerative medicine.

Changes in genomic structure and susceptibility to changes in genomic structure, i.e., genomic instability, are considered to be closely related. To eliminate the risk factor, it was necessary to select high-quality induced pluripotent stem cells with high stability of genomic structure by finally subjecting induced pluripotent stem cells after long-term culture which are to be used for clinical application to observation of cellular and nuclear morphology and tests such as karyotype analysis to observe the number and morphology of chromosomes during mitotic metaphase, whole genome sequence analysis to examine mutation of gene, SNP analysis which is Single Nucleotide Polymorphism analysis, CNV analysis which is Copy Number Variation analysis and so on (non-patent document 1) .

### [Document List]

### [non-patent document]

non-patent document 1: Martins-Taylor K., Xu R.H.: Concise review: Genomic stability of human induced pluripotent stem cells. Stem Cells 30(1): p22-27, 2012.

### [SUMMARY OF THE INVENTION]

### [Problems to be Solved by the Invention]

However, since the above-mentioned tests are time consuming and expensive, provision of a new evaluation method of induced pluripotent stem cells, a selection method of an induced pluripotent stem cell having high stability of the genomic structure and low risk of tumorigenesis, a production method of an induced pluripotent stem cell having high stability of the genomic structure and low risk of tumorigenesis has been the problem for promoting regenerative medicine using induced pluripotent stem cells.

An object of the present invention is to provide a new evaluation method of induced pluripotent stem cells that can be performed rapidly at a low cost as compared with conventional methods, a selection method of an induced pluripotent stem cell having high stability of the genomic structure and low risk of tumorigenesis, a production method of an induced pluripotent stem cell having high stability of the genomic structure and low risk of tumorigenesis, and an induced pluripotent stem cell having high stability of the genomic structure and low risk of tumorigenesis.

### [Means of Solving the Problems]

Accordingly, the present invention provides the following [1] to [24].
[1] A method for evaluating induced pluripotent stem cells, comprising the following steps (1) to (3):
   (1) a step of providing cultured induced pluripotent stem cells,
   (2) a step of measuring a spontaneous frequency of an organelle having an abnormal nucleic acid structure in the provided induced pluripotent stem cells, and
   (3) a step of identifying induced pluripotent stem cells having the aforementioned spontaneous frequency of not more than a reference value, and induced pluripotent stem cells having the aforementioned spontaneous frequency exceeding the reference value.
[2] The evaluation method of [1], wherein the aforementioned organelle having the abnormal nucleic acid structure is micronucleus.
[3] The evaluation method of [1] or [2], wherein the reference value of the aforementioned spontaneous frequency is a statistical value of the spontaneous frequency in embryonic stem cells.
[4] The evaluation method of [3], wherein the aforementioned embryonic stem cells are human embryonic stem cells.
[5] The evaluation method of [1] or [2], wherein the reference value of the aforementioned spontaneous frequency is a statistical value of the spontaneous frequency in induced pluripotent stem cells to be the reference.
[6] The evaluation method of [2], wherein the reference value of the aforementioned spontaneous frequency is 2%.
[7] The evaluation method of any of [1] to [6], wherein the aforementioned induced pluripotent stem cells are human induced pluripotent stem cells.
[8] A method for selecting induced pluripotent stem cells, comprising the following steps (1) to (4):
   (1) a step of providing cultured induced pluripotent stem cells,
   (2) a step of measuring a spontaneous frequency of an organelle having an abnormal nucleic acid structure in the provided induced pluripotent stem cells,
   (3) a step of identifying induced pluripotent stem cells having the aforementioned spontaneous frequency of not more than a reference value, and induced pluripotent stem cells having the aforementioned spontaneous frequency exceeding the reference value, and
   (4) a step of selecting induced pluripotent stem cells having the aforementioned spontaneous frequency of not more than a reference value.
[9] The selection method of [8], wherein the aforementioned organelle having the abnormal nucleic acid structure is micronucleus.
[10] The selection method of [8] or [9], wherein the reference value of the aforementioned spontaneous frequency is a statistical value of the spontaneous frequency in embryonic stem cells.
[11] The selection method of [10], wherein the aforementioned embryonic stem cells are human embryonic stem cells.
[12] The selection method of [8] or [9], wherein the reference value of the aforementioned spontaneous frequency is a statistical value of the spontaneous frequency in induced pluripotent stem cells to be the reference.
[13] The selection method of [9], wherein the reference value of the aforementioned spontaneous frequency is 2%.
[14] The selection method of any of [8] to [13], wherein the aforementioned induced pluripotent stem cells are human induced pluripotent stem cells.
[15] A method for producing induced pluripotent stem cells, comprising the following steps (1) to (4):
   (1) a step of providing established and cultured induced pluripotent stem cells,
   (2) a step of measuring a spontaneous frequency of an organelle having an abnormal nucleic acid structure in the provided induced pluripotent stem cells,
   (3) a step of identifying induced pluripotent stem cells having the aforementioned spontaneous frequency of not more than a reference value, and induced pluripotent stem cells having the aforementioned spontaneous frequency exceeding the reference value, and
   (4) a step of selecting induced pluripotent stem cells having the aforementioned spontaneous frequency of not more than a reference value.
[16] The production method of [15] further comprising the following steps before the aforementioned step (1):
   (A) a step of establishing an induced pluripotent stem cell, and
   (B) a step of culturing the established induced pluripotent stem cell.
[17] The production method of [15] or [16], wherein the aforementioned organelle having the abnormal nucleic acid structure is micronucleus.
[18] The production method of any of [15] to [17], wherein the reference value of the aforementioned spontaneous frequency is a statistical value of the spontaneous frequency in embryonic stem cells.
[19] The production method of [18], wherein the aforementioned embryonic stem cells are human embryonic stem cells.
[20] The production method of any of [15] to [17], wherein the reference value of the aforementioned spontaneous frequency is a statistical value of the spontaneous frequency in induced pluripotent stem cells.
[21] The production method of [17], wherein the reference value of the aforementioned spontaneous frequency is 2%.
[22] The production method of any of [15] to [21], wherein the aforementioned induced pluripotent stem cells are human induced pluripotent stem cells.
[23] An induced pluripotent stem cell having a spontaneous frequency of micronucleus of not more than 2%.
[24] A method for evaluating induced pluripotent stem cells, comprising the following steps (1) to (3):
   (1) a step of providing cultured induced pluripotent stem cells,
   (2) a step of measuring a spontaneous frequency of an organelle having an abnormal nucleic acid structure in the provided induced pluripotent stem cells, and
   (3) a step of evaluating that induced pluripotent stem cells having the aforementioned spontaneous frequency of not more than a reference value are induced pluripotent stem cells having a higher differentiation efficiency than induced pluripotent stem cells having the aforementioned spontaneous frequency exceeding the reference value.

### [Effect of the Invention]

According to the present invention, a new evaluation method of induced pluripotent stem cells, a selection method of induced pluripotent stem cells having high stability of the genomic structure and low risk of tumorigenesis, a production method of induced pluripotent stem cells having high stability of the genomic structure and low risk of tumorigenesis, and an induced pluripotent stem cell having high stability of the genomic structure and low risk of tumorigenesis can be provided.

### [Brief Description of the Drawings]

Fig. 1 shows a specimen image of human iPS cells "2" as a representative image of normal cells, obtained by observation of nuclear morphology of human iPS cells (Example 3).
Fig. 2 shows a specimen image of human iPS cells "D-1R(-)#2" as a representative image of abnormal cells, obtained by observation of nuclear morphology of human iPS cells (Example 3). The nuclear membrane surface is not smooth but takes a polygon-like shape, and cells suspected to have damaged nuclear envelope are noticeably observed.
Fig. 3 shows a specimen image of human iPS cells "F-1R(+)#1" as a representative image of abnormal cells, obtained by observation of nuclear morphology of human iPS cells (Example 3). Multinuclear cells having two or more nuclei in one cell are noticeably observed.
Fig. 4 shows a specimen image of human iPS cells "9" as a representative image of abnormal cells, obtained by observation of nuclear morphology of human iPS cells (Example 3). Cells with a protrusion structure from the nuclear envelope are noticeably observed.

### [Description of Embodiments]

The description of embodiments is explained in detail below.

### <Explanation of common terms>

Unless particularly specified, the terms commonly used in the present specification mean the following.

The "organelle having an abnormal nucleic acid structure" means an organelle having an abnormal structure consisting of nucleic acids, which is formed as derived from chromosomal aberration and found in cells of a mitotic anaphase to interphase or quiescent phase. Specifically, micronucleus, NPB (Nucleoplasmic bridge) and NBUD (Nuclear bud) can be mentioned.

The "spontaneous frequency of organelle having an abnormal nucleic acid structure" (hereinafter sometimes to be indicated as the spontaneous frequency) means a frequency of organelle having an abnormal nucleic acid structure, which is measured under a condition without any special treatment that may influence formation of the organelle having an abnormal nucleic acid structure, during general maintenance culture and so on. The frequency of organelle having an abnormal nucleic acid structure can be determined by calculating the proportion of cells having organelle having an abnormal nucleic acid structure relative to the total cells.

The "micronucleus" means an organelle with an abnormal nucleic acid structure that is smaller than the main nucleus, which is found in the cytoplasm of a cell in mitotic anaphase to interphase or quiescent phase. The micronucleus is known to be caused by an acentric chromosome fragment originated from structural aberration of chromosome or whole chromosome that could not move to the pole due to abnormality of chromosomal segregation in mitotic phase, and becomes an index of chromosomal aberration developed during mitosis. Micronuclear frequency shows a frequency of occurrence of structural or numerical aberration in chromosome due to cell division during a certain culture period.

The "spontaneous frequency of micronucleus", that is, frequency of spontaneous micronucleus, means a micronuclear frequency which is measured under a condition without any special treatment that may influence formation of micronucleus, such as exposure of a micronucleus inducing factor to the cell and so on, during general maintenance culture and so on. It is known that DNA replication errors and chromosomal distribution abnormalities probabilistically occur during the normal mitosis process even without exposure to micronucleus inducing factors, and so on, which in turn causes natural development of chromosomal aberration and formation of micronucleus. When micronucleus is produced in cells, some of them lead to cell death and are rapidly eliminated as abnormal cells by the homeostatic function of the cells; however, the remaining cells are detected as cells having micronuclei. Therefore, the frequency of spontaneous micronucleus can be an index totally showing stability of genetic information of the cell, such as the likelihood of occurrence of various abnormalities in the cell division process that cause chromosomal aberrations, and the degree of homeostatic function that eliminates abnormalities that have once occurred.

The "stem cell" means a cell having an ability to divide and make the same cell as the original cell, that is, self-replication ability and an ability to differentiate into a different type of cell, and capable of proliferating continuously.

The "pluripotent stem cell" means a stem cell that permits cultivation in vitro, and having the potential for differentiating into tissues derived from the three primary germ layers of the embryo (ectoderm, mesoderm and endoderm), namely, pluripotency. The "pluripotent stem cell" can be established from a fertilized egg, a cloned embryo, a germ stem cell, or a stem cell in tissue. More specific examples of the "pluripotent stem cell" include embryonic stem cells (ES cells) and induced pluripotent stem cells (iPS cells) induced from somatic cells.

The "ES cell" is a stem cell having a self-replication ability and pluripotency and means a pluripotent stem cell derived from an early embryo. Embryonic stem cell was established for the first time in 1981 and has also been applied to generate knockout mouse from 1989. In 1998, human embryonic stem cell was established and has also been utilized to regenerative medicine.

The "induced pluripotent stem cell" is a pluripotent stem cell induced from a somatic cell and means a cell artificially imparted with pluripotency similar to that of embryonic stem cell by reprogramming the somatic cell. For example, an iPS cell (induced pluripotent stem cell) with multipotency established by reprogramming differentiated cells such as fibroblast and so on by expression of genes such as Oct3/4, Sox2, Klf4, Myc and so on, and so on. can be mentioned. In 2006, Yamanaka et al. established an induced pluripotent stem cell from mouse fibroblasts (Cell, 2006, 126(4), p663-676). In 2007, they established an induced pluripotent stem cell having multipotency similar to that of embryonic stem cells from human fibroblasts (Cell, 2007, 131(5), p861-872; Science, 2007, 318(5858), p1917-1920; Nat Biotechnol., 2008, 26(1), p101-106). The induced pluripotent stem cell to be used in the present invention may be produced from somatic cell by a method known per se, or may be an already established induced pluripotent stem cell. The somatic cell from which the induced pluripotent stem cell to be used in the present invention derives is not particularly limited.

The origin of the induced pluripotent stem cell is not particularly limited. For example, induced pluripotent stem cell derived from rodents such as mouse, rat, hamster, guinea pig and so on, and induced pluripotent stem cell derived from primates such as human, monkey, orangutan, chimpanzee and so on can be mentioned, and induced pluripotent stem cell derived from human is preferable.

The "karyotype analysis" is an analysis method for examining whether there are any abnormalities in the structure or number of chromosomes of cells of interest, that is, features different from those of normal karyotype, the method including culturing the cells of interest for a given period, preparing a chromosome sample from mitotic metaphase cells enriched by adding a mitosis inhibitor such as colcemid, applying a staining that can distinguish individual chromosomes in one cell, and observing each chromosome under a microscope. For karyotype analysis, classical differential staining methods such as G band method, Q band method and so on, fluorescence in situ hybridization method using chromosome specific DNA probe (FISH; Fluorescece in situ Hybridization) and so on are widely used. Reports on karyotype analysis of iPS cells are summarized in "Stem Cells 30(1), p22-27, 2012" and so on, in which karyotype abnormality was found in a part of iPS cells, and characteristic abnormalities with plural reports are known to be trisomy of chromosome 12 and so on.

The karyotype analysis is characterized in that the kind of abnormality can be identified by directly observing the morphological characteristics of chromosome structure or an increase or decrease in the number of chromosomes; however, observation of individual chromosomes requires sufficient experience. In addition, the number of cells that can be examined is limited to a very small number because observation requires a large amount of labor, and it is problematically difficult to detect infrequent abnormalities, to quantitatively estimate the frequency of development of abnormalities and to quantitatively compare them between cells. In karyotype analysis, moreover, since the characteristics of chromosomes at a certain time point are analyzed, it is difficult to quantitatively compare time-course changes associated with mitosis during culture.

The "whole genome sequence analysis" means an analysis method including fragmenting a DNA in the cells of interest, performing parallel analysis using various sequencers to comprehensively specify the entire base sequences of the DNA molecule, and detecting DNA mutation. With the background of remarkable advances in the analytical instruments and technologies, high-precision analysis using next-generation sequencers is possible at present.

The whole genome sequence analysis is an analysis method with very high resolution, which affords information relating to the below-mentioned SNPs and CNV and can detect, at a single base level, a mutation of DNA developed at a low frequency. On the other hand, the analysis problematically requires a high cost and long period of time, where it is unpractical to examine all of many established induced pluripotent stem cells and select a line with good quality.

The "SNPs analysis" means an analysis method to detect single nucleotide polymorphism (SNPs) observed in a DNA in a cell of interest. SNPs mean single base mutations in DNA which is found at not less than a certain frequency in a population of individuals. Various SNPs have heretofore been reported also in human, and relationship with individual differences such as disease susceptibility has been pointed out. For SNPs analysis, the comprehensive analysis method in the aforementioned whole genome sequence analysis and a method for rapidly detecting known SNPs by utilizing polymerase chain reaction (PCR) are known. In addition, the presence or absence of a large number of SNPs can be efficiently analyzed by utilizing an SNP array in which innumerable SNP sequence probes are arranged in a microarea. Relatively small mutations can be analyzed at high resolution by analysis using an SNP array; however, cost and period become obstacles, and it is unpractical to utilize this analysis as a selection method for a large number of induced pluripotent stem cells.

"CNV analysis" means an analysis method including detecting copy number polymorphism (CNV) found in DNA in the cell of interest. CNV means variation in the number of genes such as the presence of only one copy (deletion), or the presence of not less than 3 copies (duplication) while two genes (2 copies) are generally present per one cell, and relationship with individual differences as in SNPs showing variation in base sequences has been pointed out. A method similar to SNPs analysis can be utilized for CNV analysis, and whole genome sequence analysis, analysis method by PCR, comparative genomic hybridization (CGH) method, analysis method utilizing SNP array and so on are known. The results of SNP analysis and CNV analysis of iPS cells are summarized in "Stem Cells 30(1), p22-27, 2012." and so on, and multiple characteristic mutations are known, such as duplication of chromosome 12 short arm 12p and deletion of chromosome 17 long arm 17q 21 locus. Similar to other analysis methods, cost and period become obstacles, and it is also unpractical to utilize CNV analysis as a selection method for a large number of induced pluripotent stem cells.

The "high stability of the genomic structure and low risk of tumorigenesis" means that, as compared to induced pluripotent stem cells having spontaneous frequency exceeding below-mentioned reference value, abnormalities in the cellular or nuclear morphology are less, good results are obtained by existing genomic structure evaluation methods such as karyotype analysis, whole genome sequence analysis, SNPs analysis, or CNV analysis and so on, or the efficiency of differentiation into certain cell types is high, as a result of which the risk of differentiation into tumor cells is low. As used herein, "efficiency of differentiation into certain cell types is high" means that the differentiated cells of interest can be obtained more frequently than the differentiation efficiency by using induced pluripotent stem cells having spontaneous frequency exceeding the reference value described later. It also means that, in the existing differentiation methods where clinical application is assumed, the desired differentiated cells can be obtained more frequently than conventional differentiation efficiency. Examples of the existing differentiation methods assumed to be clinically applicable include a differentiation method into T cells, a differentiation method into mesenchymal stem cells, a differentiation method into hematopoietic stem cells, a differentiation method into erythrocytes, a differentiation method into platelets, a differentiation method into vascular endothelial cells, a differentiation method into cardiac muscle cells, a differentiation method into skeletal muscles, a differentiation method into neural stem cells, a differentiation method into cerebral cortex nerve cells, a differentiation method into cerebrum limbic system nerve cells, a differentiation method into dopamine nerve cells, a differentiation method into hepatocytes, a differentiation method into bone, a differentiation method into cartilage, a differentiation method into primordial germ cells, a differentiation method into pancreatic cells, a differentiation method into retinal cells, a differentiation method into corneal cells, a differentiation method into kidney cells, a differentiation method into alveolar epithelial cells, a differentiation method into bronchial epithelial cells, a differentiation method into the intestinal tract and so on. A method for differentiation into T cells can be appropriately selected from various known differentiation induction methods and used. Examples of the known differentiation induction method include a method for inducing T lymphocyte from human ES cell described in "Timmermans, F. et al., J. Immunol., 2009, 182, 68879-6888". In addition, for example, human iPS cell established from human peripheral blood T lymphocyte can be induced to differentiate into T lymphocyte by the method described in "Nishimura, T. et al. Cell Stem Cell, 2013, 12, 114-126".

In one embodiment, the present invention provides a method for evaluating induced pluripotent stem cells, including (1) a step of providing cultured induced pluripotent stem cells, (2) a step of measuring a spontaneous frequency of an organelle having an abnormal nucleic acid structure in the provided induced pluripotent stem cells, and (3) a step of identifying induced pluripotent stem cells having the aforementioned spontaneous frequency of not more than a reference value, and induced pluripotent stem cells having the aforementioned spontaneous frequency exceeding the reference value (hereinafter to be abbreviated as "the evaluation method of the present invention").

In another embodiment, the present invention provides a method for selecting induced pluripotent stem cells further including, after step (3) of the evaluation method of the present invention, (4) a step of selecting induced pluripotent stem cells having the aforementioned spontaneous frequency of not more than a reference value (hereinafter to be abbreviated as "the selection method of the present invention"). In this embodiment, induced pluripotent stem cells having the aforementioned spontaneous frequency of not more than a reference value can be selected as induced pluripotent stem cells having high stability of the genomic structure and low risk of tumorigenesis as compared to induced pluripotent stem cells having the spontaneous frequency exceeding the reference value.

In a still another embodiment, the present invention provides a method for producing induced pluripotent stem cells, in which step (1) of the selection method of the present invention is replaced with a step of providing established and cultured induced pluripotent stem cells (hereinafter to be abbreviated as "the production method of the present invention").

In a still another embodiment, the present invention provides a method for producing differentiated cells derived from induced pluripotent stem cells, further including, after step (4) of the production method of the present invention, (5) a step of inducing differentiation of the induced pluripotent stem cells selected in (4) to give a differentiated cell. That is, the present invention provides a method for producing differentiated cells derived from induced pluripotent stem cells, including
(1) a step of providing established and cultured induced pluripotent stem cells,
(2) a step of measuring spontaneous frequency of organelle having an abnormal nucleic acid structure in the provided induced pluripotent stem cells,
(3) a step of identifying induced pluripotent stem cells having the aforementioned spontaneous frequency of not more than a reference value, and induced pluripotent stem cells having the aforementioned spontaneous frequency exceeding the reference value, and
(4) a step of selecting induced pluripotent stem cells having the aforementioned spontaneous frequency of not more than a reference value, and
(5) a step of inducing differentiation of the induced pluripotent stem cells selected in (4) to give differentiated cells.

The aforementioned production method of differentiated cells derived from induced pluripotent stem cells can also further include the following steps before the aforementioned step (1):
(A) a step of establishing an induced pluripotent stem cell, and
(B) a step of culturing the established induced pluripotent stem cell.

In a still another embodiment, the present invention provides, as induced pluripotent stem cells having high stability of the genomic structure and low risk of tumorigenesis, induced pluripotent stem cells having a spontaneous frequency of micronucleus of not more than 2%, not more than 1.9%, not more than 1.8%, not more than 1.7%, not more than 1.6%, not more than 1.5%, not more than 1.4% or not more than 1.3%.

Each step is specifically described below.

### <a step of providing cultured induced pluripotent stem cells>

The method for establishing an induced pluripotent stem cell is not particularly limited as long as it contains a step of introducing a particular reprogramming factor into a somatic cell. For example, a step of collecting somatic cells, artificially expressing them by introducing a reprogramming factor such as Oct3/4, Sox2, Klf4, Myc and so on, selecting cells that have acquired pluripotency and subjecting them to expansion culture, a step of introducing a reprogramming factor (Oct3/4, Sox2, Klf4, and c-Myc) into a human peripheral blood lymphocyte by using a retrovirus vector or Sendaivirus vector and culturing same (Nishimura, T. et al. Cell Stem Cell 2013, 12, 114-126) and so on can be mentioned.

Examples of the somatic cells include, but are not limited to, Lymphocytes in peripheral blood, fibroblasts of skin and so on, skin cells, visual cells, brain cells, hair cells, oral mucosa, lung cells, hepatocytes, gastric mucous cells, enterocytes, splenocytes, pancreatic cells, renal cells, neural stem cells, hematopoietic stem cells, mesenchymal stem cells derived from wisdom teeth, etc., tissue stem cells, tissue progenitor cells, blood cells (e.g., peripheral blood mononuclear cells (including T cells and non-T cells), cord blood cells, etc.), epithelial cells, endothelial cells (e.g., vascular endothelial cells), muscle cells and so on.

Examples of the gene contained in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sa111, Sall4, Esrrb, Nr5a2, Tbx3, Glis1 and so on. These reprogramming factors may be used alone or in combination. Examples of the combination of the reprogramming factors include combinations described in WO 2007/069666, WO 2008/118820, WO 2009/007852, WO 2009/032194, WO 2009/058413, WO 2009/057831, WO 2009/075119, WO 2009/079007, WO 2009/091659, WO 2009/101084, WO 2009/101407, WO 2009/102983, WO 2009/114949, WO 2009/117439, WO 2009/126250, WO 2009/126251, WO 2009/126655, WO 2009/157593, WO 2010/009015, WO 2010/033906, WO 2010/033920, WO 2010/042800, WO 2010/050626, WO 2010/056831, WO 2010/068955, WO 2010/098419, WO 2010/102267, WO 2010/111409, WO 2010/111422, WO 2010/115050, WO 2010/124290, WO 2010/147395, WO 2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell Stem Cell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, Judson R.L. et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, Maekawa M, et al. (2011), Nature. 474:225-229.

As a method for introducing a reprogramming factor into a somatic cell, when the reprogramming factor is in the form of a DNA, for example, vectors of virus, plasmid, artificial chromosome and so on, methods such as lipofection, liposome, microinjection and so on, and so on. can be mentioned, when it is in the form of an RNA, for example, methods such as lipofection, microinjection and so on can be mentioned, and when the reprogramming factor is in the form of a protein, lipofection, fusion with cell penetrating peptide (e.g., TAT derived from HIV and polyarginine), microinjection and so on can be mentioned. Examples of the method using a virus vector include, but are not limited to, a method using a retrovirus vector, a method using an episomal vector, a method using Sendaivirus vector as represented by the reprogramming kit "CytoTune (registered trademark)-iPS 2.0" of ID Pharma, a method using a lentivirus vector, a method using an adenovirus vector and so on.

The method for culturing induced pluripotent stem cells is not particularly limited as long as the induced pluripotent stem cells survive or proliferate while maintaining an undifferentiated state. For example, a method including exposing cells in a medium to which various factors have been added and maintaining an undifferentiated state to ensure survival or proliferation can be mentioned. In addition, an on-feeder culture method on mitomycin C-treated mouse fibroblasts and a feederless culture method using artificial basement membrane matrix and so on can be mentioned; however, the culture method in the present invention is not limited.

Examples of the medium include, but are not limited to, Eagle's medium (e.g., DMEM, BME, MEM, αMEM), Ham's medium (e.g., F10 medium, F12 medium), RPMI medium (e.g., RPMI-1640 medium, RPMI-1630 medium), MCDB medium (e.g., MCDB 104, 107, 131, 151, 153 media), Fischer's medium, 199 medium, and commercially available culture media [primates ES cell medium (primate ES/iPS cell culture medium, Reprocell Incorporated), mouse ES cell medium (TX-WES culture medium, Thromb-X), serum-free medium (mTeSR, Stemcell Technology), ReproFF, StemSpan (registered trade mark) SFEM, StemSpan (registered trade mark) H3000, StemlineII, ESF-B medium, ESF-C medium, CSTI-7 medium etc.]. Furthermore, a mixture of these media can also be used as necessary and, for example, DMEM/F12 medium and so on can be mentioned.

The medium can be appropriately added with 10 - 20% of serum (fetal bovine serum (FBS), human serum, horse serum) or serum replacement (KSR etc.), insulin, various vitamins, L-glutamine, various amino acids such as non-essential amino acids and so on, β-mercaptoethanol, various cytokines (interleukins (IL-2, IL-7, IL-15 etc.), stem cell factor (SCF), activin etc.), various hormones, various growth factors (leukemia inhibitory factor (LIF), basic fibroblast growth factor (bFGF), TGF-β etc.), various extracellular matrices, various cell adhesion molecules, antibiotics such as penicillin/streptomycin, puromycin and so on, pH indicators such as Phenol red and so on, and so on.

Culturing can be performed, for example, under 1 - 10%, preferably 2 - 5% CO₂-containing atmosphere at 30 - 40°C, preferably 37 - 38.5°C, for about 25 - 50 days.

To ensure a sufficient number of cells to be observed for the measurement of the frequency of spontaneous micronucleus with high accuracy, induced pluripotent stem cells are expansion-cultured to not less than 1×10³ cells, preferably not less than 1×10⁴ cells, more preferably not less than 5×10⁴ cells, whereby the frequency of spontaneous micronucleus can be efficiently measured.

The form of providing the thus-obtained "cultured induced pluripotent stem cells" or "established and cultured induced pluripotent stem cells" is not particularly limited. For example, cultured cells may be provided in a culture medium, or the cells may be provided in a cryopreserved state. Alternatively, the cells may be provided in a fixed state on a culture dish or prepared slide (sampled state). The source and destination of the cells may be the same or different.

### <Step of measuring spontaneous frequency of organelle having an abnormal nucleic acid structure>

The method for measuring the spontaneous frequency of organelle having an abnormal nucleic acid structure in the present invention is not limited and various known methods can be utilized.

The measurement of the spontaneous frequency of organelle having an abnormal nucleic acid structure which is a micronucleus is mainly described in the following. The organelle having an abnormal nucleic acid structure is not limited to micronucleus.

The following methods can be recited as the measurement of the frequency of spontaneous micronucleus. The cells after culturing are recovered, the medium is substituted by a cell fixation solution and the cells are fixed. The cell suspension after fixation are prepared to a cell density at which the suspension becomes slightly cloudy and the suspension was added dropwise on a slide glass and air dried to prepare a micronucleus specimen. The micronucleus specimen is stained, and the cells are observed under a microscope provided with an optical system compatible with the staining method. As described below, an inhibitor of cytokinesis may be added before recovery of the cells. When an inhibitor of cytokinesis is not used, the frequency of spontaneous micronucleus can be determined by measuring the number of cells without the micronucleus and the number of cells having of the micronucleus and calculating the proportion of the number of cells having of the micronucleus to the total cells observed. When an inhibitor of cytokinesis is used, the frequency of spontaneous micronucleus can be determined by measuring the number of binucleate cells and the number of binucleate cells having the micronucleus, and calculating the proportion of the binucleate cells having the micronucleus to the total binucleate cells.

The composition of the cell fixation solution used for preparation of a micronucleus specimen is not particularly limited, and ethanol, methanol, a mixture of ethanol and acetic acid, a mixture of methanol and acetic acid, a dilution solution of para-formaldehyde and so on can be mentioned.

Prior to fixation, a hypotonic treatment may be performed to favorably maintain the cytoplasm and facilitate observation. The composition of the hypotonic solution is not particularly limited and, for example, 75 mM KCl solution and so on can be mentioned.

The method for staining a micronucleus specimen is not particularly limited and, for example, double staining method of nucleus and cytoplasm with acridine orange and Giemsa staining method, nuclear staining method utilizing DAPI (2-(4-amidinophenyl)-1H-indole-6-carboxamidine), Hoechst 33342, Hoechst 33258, PI (Propidium Iodide), ethidium bromide, SYBR (registered trade mark) Gold, SYBR (registered trade mark) Green or other nucleic acid staining reagents, and so on can be mentioned.

While the number of cells to be observation object is not particularly limited, not less than 1000 object cells are generally observed to ensure statistical accuracy. The observation step of the micronucleus specimen can also be automated by using an image analyzer such as imaging cytometer and so on. In addition, to facilitate testing of many samples, it is also possible to fix the cells directly on a culture vessel such as a 96-well plate without collecting the cells and prepare a micronucleus specimen on the surface of the culture vessel. Using the aforementioned image analyzer, high-throughput automatic analysis can be performed. As an application example of the image analyzer, "Mutat Res 2013, 751(1), p1-11" and so on have been reported but it is not limited thereto. Alternatively, it is also possible to appropriately stain the recovered cell suspension and automatically analyze a large number of cells by using a cell analyzer in a flow path system such as a flow cytometer or a laser scanning cytometer. As a utilization example of the flow cytometer, "Mutat Res 2010, 703(2), p191-199" and so on have been reported but it is not limited thereto.

For the measurement of the frequency of spontaneous micronucleus, the spontaneous frequency of organelle other than the micronucleus such as NBUD and so on may also be measured in addition to micronucleus. For the purpose of obtaining information relating to mitotic dynamics and so on, an inhibitor of cytokinesis may be added before cell recovery. The addition of an inhibitor of cytokinesis also enables measurement of the spontaneous frequency of NPB. The inhibitor of cytokinesis is used at a concentration and a treatment time that does not cause noticeable cytotoxicity. Examples of the inhibitor of cytokinesis include, but are not limited to, 3 - 6 µg/mL of cytochalasin B dilution solution and so on.

As the method for measuring the spontaneous frequency of NPB, a method same as the measurement method for frequency of spontaneous micronucleus when an inhibitor of cytokinesis is used can be utilized, but the method is not limited thereto.

### <Step of identifying induced pluripotent stem cells having the spontaneous frequency of not more than a reference value, and induced pluripotent stem cells having the spontaneous frequency exceeding the reference value>

In this step, induced pluripotent stem cells for which the spontaneous frequency was measured are identified based on whether the spontaneous frequency was not more than a reference value or the spontaneous frequency was above the reference value.

As used herein, the "reference value" can be determined based on the spontaneous frequency for ES cell known to have high safety. It can also be determined based on the statistical value of the spontaneous frequency in plural ES cell lines. For example, the mean of spontaneous frequency is calculated for each ES cell line by performing the experiment multiple times, the mean of the spontaneous frequency is compared among the ES cell lines, and the mean of the ES cell line for which mean of the spontaneous frequency is the highest can be adopted as the reference value. In this case, a preferable reference value is, for example, 2%. Alternatively, the maximum value of the mean of spontaneous frequency is calculated for each ES cell line by performing the experiment multiple times, the maximum value of the mean of the spontaneous frequency is compared among the ES cell lines and the maximum value of the ES cell line having the highest maximum value can be adopted as the reference value.

Alternatively, the "reference value" can also be determined based on the statistical value of the spontaneous frequency in the iPS cell to be the standard. For example, as regards particular iPS cell lines confirmed to have high safety by an existing quality evaluation index, the mean of the spontaneous frequency is calculated by performing the experiment multiple times, the mean of the spontaneous frequency is compared among the iPS cell lines, and the mean of the iPS cell line for which mean of the spontaneous frequency is the highest can be adopted as the reference value. Alternatively, the maximum value of the spontaneous frequency is calculated for each iPS cell line by multiple times of experiment, the maximum value of the spontaneous frequency is compared among iPS cell lines, and the maximum value of the iPS cell line having the highest maximum value can be adopted as the reference value. In addition, the 50 percentile value (median value) of the spontaneous frequency of iPS cell line may be adopted as the reference value, or the 30 percentile value may be adopted as the reference value, or the mean may be adopted as the reference value. To be specific, as regards a particular iPS cell lines composed of multiple cell lines, the mean of spontaneous frequency is calculated by performing the experiment multiple times, and a median value, a 30 percentile value or mean of the means of the spontaneous frequency in the iPS cell lines or others can be adopted as the reference value.

Then, induced pluripotent stem cells having the spontaneous frequency of not more than the reference value can be identified as induced pluripotent stem cells having high stability of the genomic structure and low risk of tumorigenesis.

As shown in the below-mentioned Examples, it was shown that induced pluripotent stem cells having the spontaneous frequency of not more than a reference value has a high differentiation efficiency as compared to induced pluripotent stem cells having the spontaneous frequency exceeding the reference value. Therefore, in another embodiment of the present invention, an evaluation method of induced pluripotent stem cells is provided in which the aforementioned identification step is replaced with a step of evaluating that induced pluripotent stem cells having spontaneous frequency of not more than a reference value are induced pluripotent stem cells having a higher differentiation efficiency than induced pluripotent stem cells having the spontaneous frequency exceeding the reference value.

### <Step of selecting induced pluripotent stem cells having spontaneous frequency of not more than the reference value>

In this step, induced pluripotent stem cells having the spontaneous frequency of not more than the reference value are selected as induced pluripotent stem cells having high stability of the genomic structure and low risk of tumorigenesis. By evaluation of the genomic structure or measurement of differentiation efficiency by an existing method, it can be confirmed that the selected induced pluripotent stem cells are induced pluripotent stem cells having high stability of the genomic structure and low risk of tumorigenesis. In addition, by selecting induced pluripotent stem cells by setting the reference value of the frequency of spontaneous micronucleus to not more than 2%, not more than 1.9%, not more than 1.8%, not more than 1.7%, not more than 1.6%, not more than 1.5%, not more than 1.4% or 1.3%, an induced pluripotent stem cells having high stability of the genomic structure and low risk of tumorigenesis can be obtained.

### <Step of inducing differentiation of the selected induced pluripotent stem cells to give differentiated cells>

A method for inducing differentiation of induced pluripotent stem cells selected in the present invention is not limited and various known differentiation methods can be utilized. Examples of known differentiation methods include a differentiation method into T cells, a differentiation method into mesenchymal stem cells, a differentiation method into hematopoietic stem cells, a differentiation method into erythrocytes, a differentiation method into platelets, a differentiation method into vascular endothelial cells, a differentiation method into cardiac muscle cells, a differentiation method into skeletal muscles, a differentiation method into neural stem cells, a differentiation method into cerebral cortex nerve cells, a differentiation method into cerebrum limbic system nerve cells, a differentiation method into dopamine nerve cells, a differentiation method into hepatocytes, a differentiation method into bone, a differentiation method into cartilage, a differentiation method into primordial germ cells, a differentiation method into pancreatic cells, a differentiation method into retinal cells, a differentiation method into corneal cells, a differentiation method into kidney cells, a differentiation method into alveolar epithelial cells, a differentiation method into bronchial epithelial cells, a differentiation method into the intestinal tract and so on. As a method for differentiation into T cells, it can be appropriately selected from various known differentiation induction methods and used. Examples of the known differentiation induction method include a method for inducing T lymphocyte from human ES cell described in "Timmermans, F. et al., J. Immunol., 2009, 182, 68879-6888". In addition, for example, human iPS cell established from human peripheral blood T lymphocyte can be induced to differentiate into T lymphocyte by the method described in "Nishimura, T. et al. Cell Stem Cell, 2013, 12, 114-126".

The present invention is explained in more detail in the following by referring to Examples, which are not to be construed as limitative.

### [Examples]

### Reference Example 1 Measurement of frequency of spontaneous micronucleus of human ES cell

### (1) Human ES cell

The frequency of spontaneous micronucleus of 5 kinds of human ES cell lines KhES-1, KhES-2, KhES-3, KhES-4 and KhES-5, provided by the Institute for Frontier Medical Sciences, Kyoto University, was measured. According to the methods described in "Ueno, M. et al. PNAS 2006, 103(25), 9554-9559", "Watanabe, K. et al. Nat Biotech 2007, 25, 681-686", human ES cells were seeded on mouse fibroblasts (REPROcell) treated with mitomycin C, and maintained for culture under 37°C, 2% CO₂ conditions. As the medium therefor, DMEM/F12 medium (Sigma-Aldrich) supplemented with 20% KnockOut™ Serum Replacement (KSR, Invitrogen), 0.1 mM non-essential amino acid (NEAA; Invitrogen), 2 mM L-glutamine (Sigma-Aldrich), and 0.1 mM 2-mercaptoethanol (Wako) (hereinafter to be indicated as hES medium), and supplemented with bFGF (Wako) (10 ng/ml for KhES-1, 30 ng/ml for KhES-2, 15 ng/ml for KhES-3, 20 ng/ml for KhES-4 and KhES-5) was used. Y-27632 (Wako) (10 µM) was further added to the above-mentioned medium and used for KhES-2, KhES-3, KhES-4 and KhES-5. The medium was changed every day from one day after the start of the maintenance culture to a medium having the same composition except that Y-27632 was not contained.

The human ES cells subjected to a maintenance culture and being adhered to the cell culture dish were washed twice with phosphate-buffered saline (PBS, Invitrogen), PBS supplemented with 0.25% trypsin (Invitrogen), 1 mg/ml collagenaseIV (Invitrogen), 20% KSR, and 1 mM CaCl₂ (Nacalai Tesque) was added to the dish, and the cells were incubated under 37°C, 2% CO₂ conditions for 5 min. hES medium was added to the aforementioned dish, the cells were detached by pipetting, and the medium containing the cells was recovered and centrifuged (1000 rpm, 3 min). The supernatant was removed from the aforementioned centrifuged culture, hES medium supplemented with Y-27632 (Wako) (20 µM) was added to the precipitate to suspended the cell aggregate, and the obtained cell aggregate suspension was seeded on a cell culture dish (BD Falcon) coated with 0.1% gelatin (Sigma-Aldrich) and incubated under 37°C, 2% CO₂ conditions for 2 hr. The human ES cell mass not adhered to the dish was recovered together with the medium to give a human ES cell mass suspension free of mouse fibroblast. The suspension was centrifuged (1000 rpm, 1 min), the supernatant was removed, TrypLE™ Express (Invitrogen) supplemented with Y-27632 (20 µM) was added, and the cells were incubated at 37°C in a hot-water bath for 5 min. After dispersing the cells into single cells by pipetting and hES medium was added to give a cell suspension dispersed into single cells.

### (2) Measurement method and results of frequency of spontaneous micronucleus

The cell suspension obtained in (1) was centrifuged (1000 rpm, 5 min) and the supernatant was substituted with phosphate-buffered saline (PBS, NISSUI PHARMACEUTICAL). The obtained cell suspension was centrifuged again (1000 rpm, 5 min), the supernatant was removed, 75 mM KCl (Wako) heated to 37°C was added and a hypotonic treatment was performed for 5 min. To the cell suspension after the hypotonic treatment was added 1/5 volume of a fixation solution (methanol (nacalai tesque):glacial acetic acid (nacalai tesque)=3:1), and the cells were semi-fixed and centrifuged (4°C, 1000 rpm, 5 min). After centrifugation, the supernatant was substituted with a fresh fixation solution, and the cells were fixed and centrifuged (4°C, 1000 rpm, 5 min). The supernatant was removed, and the residue was resuspended in methanol to a cell density at which the suspension became slightly cloudy and the suspension was added dropwise on a slide glass and air dried to give a micronucleus specimen. The micronucleus specimen was stained with 100 µg/mL Acridine Orange (Wako) solution, and the cells were observed under a fluorescence microscope using a wide-band Blue excitation filter. Two glass slides for a cell line were observed, and the number of cells having the micronucleus in 1000 human iPS cells per one glass slide, namely, 2000 human iPS cells per one cell line, was measured, and the frequency of spontaneous micronucleus was calculated. The results are shown in Table 1. From Table 1, the highest frequency of spontaneous micronucleus was 2%. Since ES cells have a low risk of tumorigenesis and are considered to be safe, this 2% can be adopted as a reference value.

**[Table 1]**

| Frequency of spontaneous micronucleus of human ES cell | | |
|---|---|---|
| human ES cell line | number of passage from establishment at the time of measurement | frequency of spontaneous micronucleus |
| KhES-1 | 36 | 1.05% |
| KhES-2 | 30 | 2.00% |
| KhES-3 | 26 | 1.20% |
| KhES-4 | 37 | 1.50% |
| KhES-5 | 35 | 1.60% |

### Example 1 Measurement of frequency of spontaneous micronucleus and differentiation efficiency of human iPS cell

### (1) Human iPS cell

The frequency of spontaneous micronucleus and differentiation efficiency were measured for the human iPS cells of total 7 human iPS cell lines of "2", "3" "5" "9" "10", "11" and "12" established in Kaneko Laboratory, Center for iPS Cell Research and Application, Kyoto University, from human peripheral blood lymphocytes according to the method described in "Nishimura, T. et al. Cell Stem Cell 2013, 12, 114-126".

### (2) Measurement method of frequency of spontaneous micronucleus

According to the method described in "Nishimura, T. et al. Cell Stem Cell 2013, 12, 114-126", the human iPS cells described in (1) were seeded on mouse fibroblasts (REPROcell) treated with mitomycin C, and maintained for culture under 37°C, 2% CO₂ conditions. As the medium therefor, DMEM/F12 medium (Sigma-Aldrich) added with 20% KSR, 2 mM L-glutamine, 1% NEAA and 10 µM 2-mercaptoethanol and supplemented with bFGF 5 ng/ml was used.

Using Trypsin-EDTA (Sigma-Aldrich), only mouse fibroblasts were removed from the human iPS cells after maintenance culture, the human iPS cells were suspended in D-PBS (2% FBS) and used as a sample for the measurement of the frequency of spontaneous micronucleus. Using the cell suspension, a micronucleus specimen was produced according to the method described in Reference Example 1(2) and observed. The number of cells having the micronucleus in 1000 human iPS cells per one glass slide, namely, 2000 human iPS cells per one cell line, was measured, and the frequency of spontaneous micronucleus was calculated. The results are shown in Table 2.

From Reference Example 1, when the reference value was 2%, the human iPS cell lines of not more than the reference value were "2" and "3".

### (3) Measurement method of differentiation efficiency

The human iPS cells described in (1) were induced to differentiate into T cell lineage according to the method described in "Nishimura, T. et al. Cell Stem Cell 2013, 12, 114-126".

Immunostaining using an antibody against surface antigens CD4 and CD8 was performed, and the proportion of CD4⁺CD8⁺ cells to CD45⁺CD3⁺CD7⁺ cells was measured using a flow cytometer and used as the differentiation efficiency. The results are shown in Table 2.

**[Table 2]**

| Frequency of spontaneous micronucleus and differentiation efficiency of human iPS cell | | | |
|---|---|---|---|
| iPS cell line | measurement results of frequency of spontaneous micronucleus | | measurement results of differentiation efficiency |
| | number of passage from establishment at the time of measurement | frequency of spontaneous micronucleus | |
| "2" | 45 | 1.35% | 58.1% |
| "3" | 33 | 1.75% | 11.6% |
| "10" | 27 | 2.60% | 2.01% |
| "11" | 31 | 3.05% | 2.95% |
| "12" | 12 | 3.05% | 0.90% |
| "9" | 14 | 3.30% | 1.55% |
| "5" | 45 | 3.80% | 1.99% |

From Reference Example 1 and Example 1, the frequency of spontaneous micronucleus of human ES cells was stably low and was 1.05% - 2.00%, whereas the frequency of spontaneous micronucleus of human iPS cells was found to vary much from 1.35% to 3.80% depending on the line. This is because it is considered human ES cells are established without genetic manipulation from early embryo in the process of development, and their genomic structures are stable, whereas human iPS cells become a heterogeneous population by genetic manipulation during reprogramming and the variation in the properties including stability of genomic structure is relatively large depending on the selected cell line.

From the results of Example 1 measuring the differentiation efficiency from human iPS cell to T cell lineage, correlative relationship was found between the frequency of spontaneous micronucleus and the differentiation efficiency. When the reference value was 2%, human iPS cell lines "2" and "3" having a frequency of spontaneous micronucleus of not more than the reference value showed not less than 3 times higher differentiation efficiency as compared to a human iPS cell line having a frequency of spontaneous micronucleus exceeding the reference value.

### Example 2 Measurement of frequency of spontaneous micronucleus of T cells derived from human iPS cells

Human iPS cell lines "2", "12" were induced to differentiate into T cells which are CD8SP (Single Positive) according to the method described in "Nishimura, T. et al. Cell Stem Cell 2013, 12, 114-126". The obtained CD8SP T cells (which are hereinafter to be indicated as redifferentiated T cells) were subjected to expansion culture and the frequency of spontaneous micronucleus was measured according to the following method. That is, redifferentiated T cells were seeded on a 12 well plate (BD Falcon) coated with 1.0 µg/mL anti-CD3 antibody OKT3 (eBioscience) at a cell density of 1.0×10⁶ cells/mL to give a proliferation stimulation. As the medium at the time of seeding, RPMI-1640 medium (containing L-glutamine, Wako) added with 10% human serum AB (Nova Biologics), 1% Penicillin-Streptomycin (nacalai tesque), 100 U/mL IL-2 (Wako), 10 ng/mL IL-7 (Wako), and 10 ng/mL IL-15 (R&D SYSTEMS) (hereinafter to be indicated as Rh medium) was used. After proliferation stimulation for 17 hr, the total amount of the redifferentiated T cells were recovered, Rh medium (60 µL) was added to the cell suspension (90 µL) and the cells were reseeded on a 96 well plate (nunc) not coated with OKT3. After about 24 hr from the reseeding, the supernatant (15 µL) was removed, and Rh medium (15 µL) supplemented with 60 µg/mL cytochalasin B (Wako) was added (final concentration of cytochalasin B: 6 µg/mL). At 33 hr from the addition of cytochalasin B, the supernatant was removed, PBS was added and the mixture was stirred. After centrifugation (1500 rpm, 3 min), the supernatant was substituted with PBS again and the mixture was stirred. After centrifugation (1500 rpm, 3 min) again, the supernatant was removed, 75 mM KCl heated to 37°C was added and a hypotonic treatment was performed for 5 min. To the cell suspension after hypotonicity was added 1/5 volume of a fixation solution (methanol:glacial acetic acid=3:1), and the cells were semi-fixed and centrifuged (4°C, 1500 rpm, 3 min). After centrifugation, the supernatant was substituted with a fresh fixation solution, and the cells were fixed and centrifuged (4°C, 1500 rpm, 3 min). The supernatant was substituted again with a fresh fixation solution, and the cells were fixed and centrifuged (4°C, 1500 rpm, 3 min). Furthermore, the supernatant was substituted with methanol, and the cells were fixed and centrifuged (4°C, 1500 rpm, 3 min). The supernatant was removed, and the residue was resuspended in methanol to a cell density at which the suspension became slightly cloudy and the suspension was added dropwise on a slide glass and air dried to give a micronucleus specimen. The micronucleus specimen was stained with 40 µg/mL Acridine Orange solution, and the cells were observed under a fluorescence microscope using a wide-band Blue excitation filter. 1000 binucleate cells were observed in line "2", 1500 binucleate cells were observed in line "12", the number of binucleate cells having micronucleus was measured, and the proportion of the binucleate cells having micronucleus to the total binucleate cells was calculated. The results are shown in Table 3. Table 3 additionally shows the frequency of spontaneous micronucleus measured in Example 1.

**[Table 3]**

| Frequency of spontaneous micronucleus in redifferentiated T cells | | |
|---|---|---|
| source iPS cell line | frequency of spontaneous micronucleus in redifferentiated T cells | frequency of spontaneous micronucleus in iPS cells |
| "2" | 1.80% | 1.35% |
| "12" | 3.33% | 3.05% |

The frequency of spontaneous micronucleus of human iPS cell is also correlated with the frequency of spontaneous micronucleus of T cells after differentiation, and the possibility was suggested that a differentiated cell having high stability of the genomic structure and low risk of tumorigenesis can be obtained by selecting the human iPS cell line by the selection method of the present invention.

### Example 3 Measurement of frequency of spontaneous micronucleus of human iPS cell, karyotype analysis and observation of nuclear morphology

### (1) Human iPS cell

The frequency of spontaneous micronucleus was measured for the human iPS cells of total 5 human iPS cell lines of "D-1R(-)#1", "D-1R(-)#2", "F-1R(-)#2", "F-1R(+)#1" and "F-3R(+)#1" established in Kaneko Laboratory, Center for iPS Cell Research and Application, Kyoto University, from human peripheral blood lymphocytes by using "CytoTune (registered trade mark)-iPS 2.0" (ID Pharma).

In addition, the nuclear morphology was observed for total 12 human iPS cell lines consisting of the above-mentioned 5 lines in addition to the human iPS cell 7 lines described in Example 1, and karyotype analysis was performed for total 11 human iPS cell lines excluding "F-1R(+)#1" from the 12 lines.

### (2) Measurement of frequency of spontaneous micronucleus

Five lines of human iPS cells subjected to the measurement of frequency of spontaneous micronucleus described in (1) were seeded on iMatrix (Nippi) and maintained for culture under 37°C, 2% CO₂ conditions according to the method described in "Establishment and maintenance culture of human iPS cell at feeder free (from CiRA website)". As the medium therefore, Stemfit AK03N (Ajinomoto Co., Inc.) was used.

Human iPS cells subjected to the maintenance culture were treated with 0.5×TriPLE (trade mark of a commercial product) Select (ThermoFisher SCIENTIFIC) to prepare the single cell suspension, suspended in Stemfit AK03N and used as a sample for the measurement of the frequency of spontaneous micronucleus. The cell suspension was centrifuged (1000 rpm, 3 min) and the supernatant was substituted with phosphate-buffered saline (PBS, NISSUI PHARMACEUTICAL). The cell suspension was centrifuged again (1000 rpm, 3 min), the supernatant was removed, 75 mM KCl (Wako) heated to 37°C was added and a hypotonic treatment was performed for 5 min. To the cell suspension after the hypotonicity was added 1/5 volume of a fixation solution (methanol (nacalai tesque):glacial acetic acid (nacalai tesque)=3:1), and the cells were semi-fixed and centrifuged (4°C, 1000 rpm, 3 min). After centrifugation, the supernatant was substituted with a fresh fixation solution, and the cells were fixed and centrifuged (4°C, 1000 rpm, 3 min). The supernatant was removed, and the residue was resuspended in a 2% fixation solution (methanol:glacial acetic acid=98:2) to a cell density at which the suspension became slightly cloudy and the suspension was added dropwise on a slide glass and air dried to give a micronucleus specimen. The micronucleus specimen was stained with 50 µg/mL Acridine Orange solution, and the cells were observed under a fluorescence microscope using a wide-band Blue excitation filter. Two glass slides for a cell line were observed, and the number of cells having the micronucleus in 1000 human iPS cells per one glass slide, namely, 2000 human iPS cells per one cell line, was measured, and the frequency of spontaneous micronucleus was calculated. The results are shown in Table 4 together with the results of the measurement of the frequency of spontaneous micronucleus in Example 1(2).

### (3) Observation of nuclear morphology

A micronucleus specimen on a slide glass of 5 lines of human iPS cells prepared in (2), and a micronucleus specimen on a slide glass of 7 lines of human iPS cells produced in Example 1(2) were stained with Acridine Orange solution as in the measurement of the frequency of spontaneous micronucleus, and the nuclear morphology was observed under a fluorescence microscope using a wide-band Blue excitation filter.

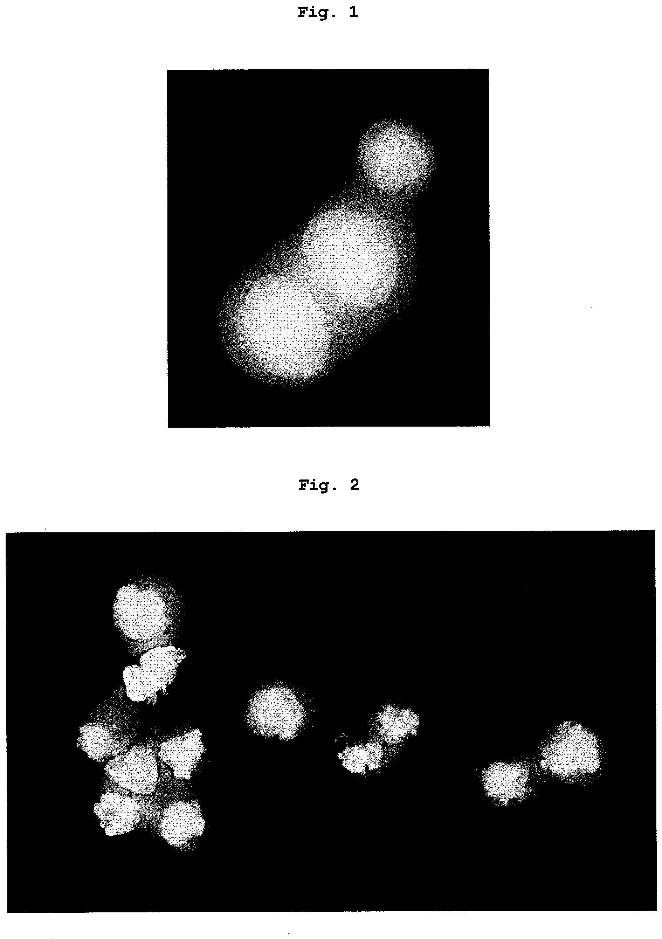

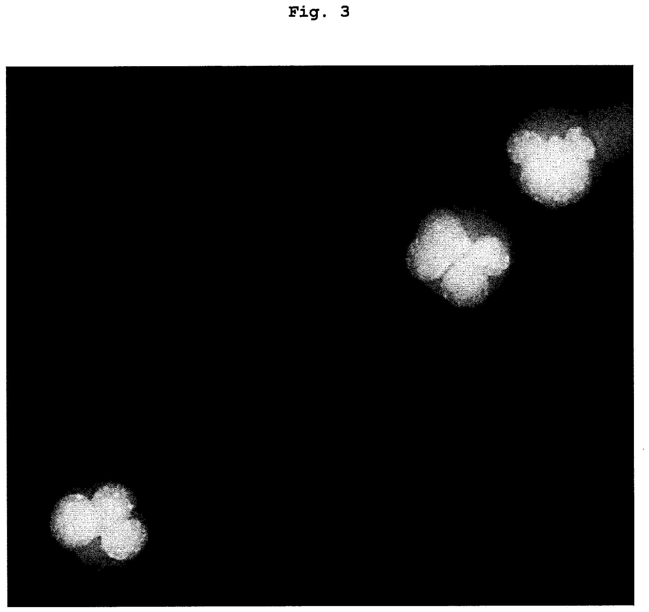

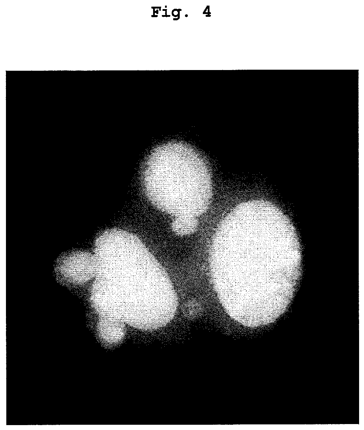

For nuclear morphology observation, cells that satisfy the following [1] and [2] were determined as "normal cells", cells that do not satisfy either [1] or [2] or both [1] and [2] were determined as "abnormal cells", a human iPS cell line in which majority of cells in the specimen are normal cells was determined as "normal" line, and a human iPS cell line noticeably having abnormal cells in the specimen was determined as "abnormal" line. The results are shown in Table 4. Images of the representative normal cells or abnormal cells are shown in Fig. 1 - Fig. 4.
[1] Outer edge of nuclear envelope is clear and one cell has one nucleus.
[2] Damage or abnormal structure such as protrusion is not found in nuclear envelope and the shape is smoothly circular or ellipsoidal.

### (4) Karyotype analysis

The human iPS cells of the total 11 lines described in (1) were subjected to maintenance culture similar to that of (2) or Example 1(2), recovered, made into a cell suspension and used as a karyotype analysis sample. The karyotype analysis was entrusted to LSI Medience Corporation. A chromosome specimen in the mitotic phase was produced from the cell suspension, subjected to differential staining by the G band method, chromosomes were classified by chromosome number, and 20 cells per human iPS cell line were analyzed.

In karyotype analysis, cells having chromosomal structural aberrations (deletion, insertion, inversion, translocation, abnormal structures of chromosome accompanying chromosome cleavage) and/or numerical aberrations (number of chromosomes different from 46 in 2 pairs such as monosomy, trisomy, tetraploid) were determined as "abnormal cells" and cells not showing any abnormality were determined as "normal cells", and a human iPS cell line in which all 20 analyzed cells are normal cells was determined as a "normal" line, and a human iPS cell line having one or more abnormal cells was determined as an "abnormal" line. The results are shown in Table 4.

**[Table 4]**

| Frequency of spontaneous micronucleus, nuclear morphology observation results and karyotype analysis results of human iPS cells | | | |
|---|---|---|---|
| iPS cell line | frequency of spontaneous micronucleus | nuclear morphology observation results | karyotype analysis results |
| "2" | 1.35% | normal | normal |
| "3" | 1.75% | normal | normal |
| "F-3R(+)#1" | 1.75% | normal | normal |
| "F-1R(-)#2" | 1.85% | normal | normal |
| "D-1R(-)#2" | 2.55% | abnormal ¹⁾ | abnormal ⁴⁾ |
| "10" | 2.60% | abnormal ²⁾ | normal |
| "11" | 3.05% | abnormal ¹⁾ | normal |
| "12" | 3.05% | normal | abnormal ⁵⁾ |
| "F-1R(+)#1" | 3.15% | abnormal ²⁾ | - |
| "9" | 3.30% | abnormal ³⁾ | normal |
| "5" | 3.80% | normal | normal |
| "D-1R(-)#1" | 4.35% | abnormal ¹⁾ | normal |

| | | | |
|---|---|---|---|
| 1) The surface of the nuclear envelope was not smooth but had a polygon-like shape, and cells with suspected nuclear envelope damage were notably observed. 2) Multinuclear cells having two or more nuclei in one cell were notably observed. 3) Cells with protrusion structure from the nuclear envelope were notably observed. 4) Chromosomal deletion was found in one out of 20 cells. 5) Chromosomal duplication was found in one out of 20 cells. | | | |

A correlative relationships was found between the frequency of spontaneous micronucleus and the karyotype or nuclear morphology showing the presence or absence of abnormality in the genomic structure. When the reference value was 2%, the karyotype and nuclear morphology of all 4 human iPS cell lines having a frequency of spontaneous micronucleus of not more than the reference value were normal, whereas at least one of the karyotype and nuclear morphology was abnormal in 7 out of 8 human iPS cell lines having a frequency of spontaneous micronucleus exceeding the reference value. That is, by selecting human iPS cell line by the selection method of the present invention, the possibility of obtaining a cell having high stability of the genomic structure and low risk of tumorigenesis was suggested.

### Example 4 Production of human iPS cell having frequency of spontaneous micronucleus of not more than reference value

### (1) Human iPS cell

Human iPS cell line is established from human peripheral blood lymphocytes according to the method described in "Nishimura, T. et al. Cell Stem Cell 2013, 12, 114-126".

### (2) Measurement of frequency of spontaneous micronucleus

According to the method described in "Nishimura, T. et al. Cell Stem Cell 2013, 12, 114-126", the human iPS cells described in (1) are seeded on mouse fibroblasts (REPROcell) treated with mitomycin C, and maintained for culture under 37°C, 2% CO₂ conditions. As the medium therefor, DMEM/F12 medium (Sigma-Aldrich) added with 20% KSR, 2 mM L-glutamine, 1% NEAA and 10 µM 2-mercaptoethanol and supplemented with bFGF 5 ng/ml is used.

Using Trypsin-EDTA (Sigma-Aldrich), only mouse fibroblasts are removed from the human iPS cells after maintenance culture, the human iPS cells are suspended in D-PBS (2% FBS) and used as a sample for the measurement of the frequency of spontaneous micronucleus. Using the cell suspension, a micronucleus specimen is prepared according to the method described in Reference Example 1(2) and observed. The number of cells having the micronucleus in 1000 human iPS cells per one glass slide, namely, 2000 human iPS cells per one cell line, is measured, and the frequency of spontaneous micronucleus is calculated.

An induced pluripotent stem cell having a frequency of spontaneous micronucleus of not more than 2% and an induced pluripotent stem cell having a frequency of spontaneous micronucleus exceeding 2% are identified. By selecting the induced pluripotent stem cell having the frequency of spontaneous micronucleus of not more than 2%, the human iPS cell line is obtained as an induced pluripotent stem cell having a frequency of spontaneous micronucleus of not more than 2%.

An induced pluripotent stem cell having a frequency of spontaneous micronucleus of not more than 1.3% and an induced pluripotent stem cell having a frequency of spontaneous micronucleus exceeding 1.3% are identified. By selecting the induced pluripotent stem cell having the frequency of spontaneous micronucleus of not more than 1.3%, the human iPS cell line is obtained as an induced pluripotent stem cell having a frequency of spontaneous micronucleus of not more than 1.3%.

From such results, it is considered that an induced pluripotent stem cell having high stability of the genomic structure and low risk of tumorigenesis can be efficiently produced conveniently at a low cost by measuring the frequency of spontaneous micronucleus of each established induced pluripotent stem cell and selecting a cell line showing the same level of frequency of spontaneous micronucleus as ES cell, and possibility of clinical application of the induced pluripotent stem cell is further increased.

This application is based on a patent application No. 2016-252672 filed in Japan (filing date: December 27, 2016), the contents of which are incorporated in full herein by reference.

### [Industrial Applicability]

An induced pluripotent stem cell having high stability of the genomic structure and low risk of tumorigenesis can be efficiently produced conveniently at a low cost by measuring the frequency of spontaneous micronucleus of the established induced pluripotent stem cell and selecting a cell line showing the same level of frequency of spontaneous micronucleus as ES cell. As a result, a high-quality induced pluripotent stem cell bank can be produced more efficiently and can be utilized for research and medical treatment. In addition, the possibility of basic research and clinical application using induced pluripotent stem cells is expected to further increase, for example, selection of clinically applicable autologous induced pluripotent stem cells, which had to be given up by conventional techniques, becomes realistic.

## Claims

1. A method for evaluating induced pluripotent stem cells, comprising the following steps (1) to (3):
(1) a step of providing cultured induced pluripotent stem cells,
(2) a step of measuring a spontaneous frequency of an organelle having an abnormal nucleic acid structure in the provided induced pluripotent stem cells, and
(3) a step of identifying induced pluripotent stem cells having the spontaneous frequency of not more than a reference value, and induced pluripotent stem cells having the spontaneous frequency exceeding the reference value.

2. The evaluation method according to claim 1, wherein the organelle having the abnormal nucleic acid structure is micronucleus.

3. The evaluation method according to claim 1 or 2, wherein the reference value is a statistical value of the spontaneous frequency in embryonic stem cells.

4. The evaluation method according to claim 3, wherein the embryonic stem cells are human embryonic stem cells.

5. The evaluation method according to claim 1 or 2, wherein the reference value of the spontaneous frequency is a statistical value of the spontaneous frequency in induced pluripotent stem cells to be the reference.

6. The evaluation method according to claim 2, wherein the reference value of the spontaneous frequency is 2%.

7. The evaluation method according to any one of claims 1 to 6, wherein the induced pluripotent stem cells are human induced pluripotent stem cells.

8. A method for selecting induced pluripotent stem cells, comprising the following steps (1) to (4):
(1) a step of providing cultured induced pluripotent stem cells,
(2) a step of measuring a spontaneous frequency of an organelle having an abnormal nucleic acid structure in the provided induced pluripotent stem cells,
(3) a step of identifying induced pluripotent stem cells having the spontaneous frequency of not more than a reference value, and induced pluripotent stem cells having the spontaneous frequency exceeding the reference value, and
(4) a step of selecting induced pluripotent stem cells having the spontaneous frequency of not more than a reference value.

9. The selection method according to claim 8, wherein the organelle having the abnormal nucleic acid structure is micronucleus.

10. The selection method according to claim 8 or 9, wherein the reference value of the spontaneous frequency is a statistical value of the spontaneous frequency in embryonic stem cells.

11. The selection method according to claim 10, wherein the embryonic stem cells are human embryonic stem cells.

12. The selection method according to claim 8 or 9, wherein the reference value of the spontaneous frequency is a statistical value of the spontaneous frequency in induced pluripotent stem cells to be the reference.

13. The selection method according to claim 9, wherein the reference value of the spontaneous frequency is 2%.

14. The selection method according to any one of claims 8 to 13, wherein the induced pluripotent stem cells are human induced pluripotent stem cells.

15. A method for producing induced pluripotent stem cells, comprising the following steps (1) to (4):
(1) a step of providing established and cultured induced pluripotent stem cells,
(2) a step of measuring a spontaneous frequency of an organelle having an abnormal nucleic acid structure in the provided induced pluripotent stem cells,
(3) a step of identifying induced pluripotent stem cells having the spontaneous frequency of not more than a reference value, and induced pluripotent stem cells having the spontaneous frequency exceeding the reference value, and
(4) a step of selecting induced pluripotent stem cells having the spontaneous frequency of not more than a reference value.

16. The production method according to claim 15 further comprising the following steps before the step (1):
(A) a step of establishing an induced pluripotent stem cell, and
(B) a step of culturing the established induced pluripotent stem cell.

17. The production method according to claim 15 or 16, wherein the organelle having the abnormal nucleic acid structure is micronucleus.

18. The production method according to any one of claims 15 to 17, wherein the reference value of the spontaneous frequency is a statistical value of the spontaneous frequency in embryonic stem cells.

19. The production method according to claim 18, wherein the embryonic stem cells are human embryonic stem cells.

20. The production method according to any one of claims 15 to 17, wherein the reference value of the spontaneous frequency is a statistical value of the spontaneous frequency in induced pluripotent stem cells.

21. The production method according to claim 17, wherein the reference value of the spontaneous frequency is 2%.

22. The production method according to any one of claims 15 to 21, wherein the induced pluripotent stem cells are human induced pluripotent stem cells.

23. An induced pluripotent stem cell having a spontaneous frequency of micronucleus of not more than 2%.

24. A method for evaluating induced pluripotent stem cells, comprising the following steps (1) to (3):
(1) a step of providing cultured induced pluripotent stem cells,
(2) a step of measuring a spontaneous frequency of an organelle having an abnormal nucleic acid structure in the provided induced pluripotent stem cells, and
(3) a step of evaluating that induced pluripotent stem cells having the spontaneous frequency of not more than a reference value are induced pluripotent stem cells having a higher differentiation efficiency than induced pluripotent stem cells having the spontaneous frequency exceeding the reference value.
